# EUROPEAN PATENT SPECIFICATION

(11) **EP 1 357 788 B1**
(45) Date of publication and mention of the grant of the patent: **09.09.2009**
(21) Application number: 02705987.2
(22) Date of filing: 25.01.2002
(51) Int. Cl.: A61K 6/00

(54) **METHOD OF MANUFACTURING TOOTH WHITENING MATERIAL**
VERFAHREN ZUR HERSTELLUNG VON ZAHNBLEICHMITTELN
PROCEDE POUR LA FABRICATION DE MATIERE DE BLANCHISSEMENT DENTAIRE

(30) Priority: 25.01.2001 US 264227 P; 23.03.2001 US 81566
(43) Date of publication of application: 05.11.2003
(73) Proprietor: DENTSPLY INTERNATIONAL, INC., New York, PA 17405-0872 (US)
(72) Inventor: PETERSON, Kenneth, S., Lancaster, PA 17603 (US); SHERMANN, James, M., York, PA 17404 (US)
(74) Representative: Hartz, Nikolai
(86) International application number: PCT/US2002/002269
(87) International publication number: WO 2002/058460

(56) References cited:
- EP-A- 0 516 872
- EP-A- 0 839 517
- WO-A-01/70178

## Description

### FIELD OF THE INVENTION

The present invention relates to tooth whitening material formed from a powder component and a liquid component, that sets to an elastomeric, semi-solid texture.

### BACKGROUND OF THE INVENTION

This invention relates in general to teeth whitening materials used by a dental practitioner to whiten human teeth. For a variety of reasons it has become desirable for a person's teeth to appear bright or "white". Society places a high value on the "whiteness" of one's teeth.

A tooth is comprised of an inner dentin layer and an outer hard enamel layer that is the protective layer of the tooth. The enamel layer of a tooth is naturally an opaque white or slightly off-white color. It is this enamel layer that can become stained or discolored.

Many substances that a person confronts or comes in contact with on a daily basis can "stain" or reduce the "whiteness" of one's teeth. In particular, the foods, tobacco products and fluids that one consumes tend to stain one's teeth. These products or substances tend to accumulate on the enamel layer of the tooth and form a pellicle film over the teeth. These staining and discoloring substances can then permeate the enamel layer. This problem occurs gradually over many years, but imparts a noticeable discoloration of the enamel of one's teeth. Some pharmaceuticals, diseases and environmental factors may also have the effect of discoloring one's teeth. So long as the discolored teeth are still healthy and do not pose any health risk or problem, a product or substance that would whiten the discolored teeth would be advantageous.

It is known in the art to provide compositions containing hydrogen peroxide and to contact a patient's teeth with the composition. Prolonged contact will bleach and whiten stained teeth. Because many known peroxide compositions are viscous or even liquid, it has been a common practice to provide a dental tray molded to the patient's dentition to hold the bleaching material in place for sufficient time to effect bleaching. Without the tray, the bleaching material either flows away on its own or is degraded and removed by saliva.

Whitening compositions may be thought of as being of two types: in-office materials and take-home materials. Dental professionals may carry out the in-office treatment, while the take-home materials are designed to be administered by the patients themselves. As can be expected, the in-office treatments can be carried out using bleaching compositions having higher peroxide concentrations as compared to take-home materials. This is for the obvious safety reasons.

The dental bleaching systems heretofore known in the art have been effective in whitening teeth. However, there has been to date the requirement that the dental tray employed be closely configured to the dentition. Otherwise, the saliva effects noted above are compounded. An exemplary tooth whitening material employing a dental tray is shown for example in U.S. Pat. No. Re. 34,196. It would be desirable however, to have a dental material that does not require a dental tray. A need exists therefore, for such an improved tooth whitening material.

EP-A-0 516 872 discloses tooth bleaching compositions comprising a concentrated aqueous solution of hydrogen peroxide (at least 30%) and suspended therein a nonaqueous component which comprises 50 to 80% silica particles and may also comprise Gantrez MS-955 (mixed sodium/calcium salt of a copolymer of methyl vinyl ether/maleic anhydride). The compositions are in the form of pastes or gels and are applied directly to the teeth.

### BRIEF DISCLOSURE OF THE INVENTION

It is therefore, an object of the present invention to provide a tooth whitening composition from a solid or powder component and a liquid component. When mixed, the resulting composition will set to an elastomeric quality within about 1 to about 5 minutes.

The present invention is set out in the claims.

### BRIEF DESCRIPTION OF THE DRAWINGS

Fig. 1 is a perspective view of a dual syringe mixing apparatus suitable for use in the present invention to mix a powder component and a liquid component, and to then dispense the material as required.
Fig. 2 is a top plan, partially schematic view of a dental arch in place thereon, and employing a backing sheet.

### PREFERRED EMBODIMENTS FOR CARRYING OUT THE INVENTION

The present invention uses a two-component tooth whitening material that is intended to be used by a dental practitioner as opposed to being used by a non-professional patient. The material employs hydrogen peroxide in concentrations preferably of from about 30 to about 35 percent by weight. The material is prepackaged with a solid or powder component separate from a liquid component. The liquid component contains the hydrogen peroxide in water, and may contain other conventional components such as stabilizers, flavoring agents, or the like.

The liquid component may also comprise other conventional whitening agents such as carbamide peroxide, if desired.

The powder component preferably comprises mixed sodium/calcium salts of poly(methyl vinyl ether/maleic anhydride). Such a polymer is available for example from International Specialty Products as Gantrez. A particularly useful grade of Gantrez is Gantrez MS-955.

The two components, liquid and powder, are blended in ratios of from 20 to 80 parts by weight of hydrogen peroxide to 20 to 80 parts by weight of Gantrez. Preferably, the Gantrez is mixed with titanium dioxide. Preferred Gantrez and titanium dioxide mixtures include from about 1 to about 99 parts by weight of Gantrez to 99 to about 1 part by weight of titanium dioxide.

One preferred embodiment of the present invention includes from about 40 to about 60 parts by weight of hydrogen peroxide and from about 60 to about 40 parts by weight of a 99:1 Gantrez/titanium dioxide powder mixture. The two parts are blended together for about 10 to about 60 seconds, and the resultant mixture is a creamy white to off-white material. The material sets to an elastomeric or rubber-like, solid or semi-solid texture in from about I to about 5 minutes. Before setting, the material can be placed into a dental tray, or applied directly to dentition without the use of a tray. Once the material has set, the tray, if used, can be left in place or removed.

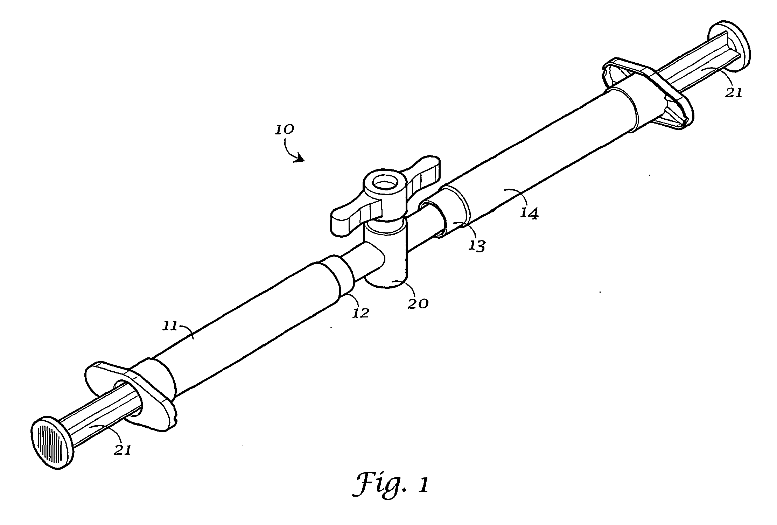

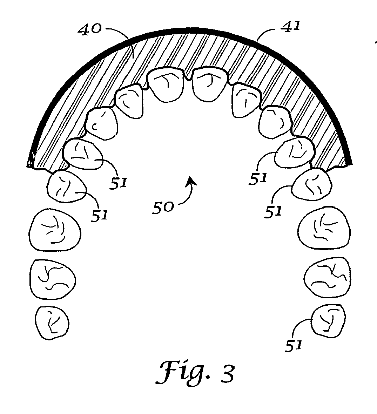

Fig. 1 shows a preferred form of packaging for materials used in the present invention. The package 10 of Fig. 1 includes a first syringe 11 connected at its output end 12 to the output end 13 of a second syringe 14. One of the other syringe 11 or 14 holds the powder component as described above, while the other holds the liquid component. Any method of selectively preventing fluid communication between syringes 11 and 14 is within the scope of the invention. For example, stopcock 20 may be employed. In this way, the materials may be packaged separately, delivered and shelved until needed. When needed, the interiors of syringes 11 and 14 are caused to come into fluid communication, such as by using otherwise conventional stopcock 20. Stopcocks such as stopcock 20 are well known in the art and the internal function of stopcock 20 need not be specifically described.

Once the interior of syringe 11 is in fluid communication with the interior of syringe 14, the user manually depresses one then the other plungers 21 of syringes 11 and 14. The contents of each syringe 11 and 14 are thereby caused to alternately flow therebetween, effecting mixing.

When mixing is completed, the syringes are separated with the mixture held in one syringe 11 or 14. That syringe 11 or 14 is then used to dispense the mixture as required.

According to Fig. 2, the whitening material 40, such as that described above is mixed such as by shaking in a container or by the use of syringes 11 and 14. Before the material has set as described above, it is decanted into a mold to form any desired shape, such as a rectangular strip as shown in the drawings, or any other shape, and allowed to at least partially set. A release or backing sheet 41 is then pressed to the at least partially set material 40. Sheet 41 can be of any material, but preferably is flexible enough to bend around the curvature of the dentition as is depicted in Fig. 2, but not so flexible that it will not physically support the material 40.

The combined sheet 41 and material 40 is then placed upon the dentition 50 having teeth 51 to be whitened, such that the material 40 is caused top contact the desired tooth or teeth portions. It will be appreciated that the material 40 will fill any interstitial gaps between individual teeth, as well as any cracks, fissures or other dentition profiles, as is also depicted in the drawings. Sheet 41 does not conform to the shape of a given tooth, although it does curve to allow proper placement of the material 40 as described. When the treatment is finished, sheet 41 and material 40 can be simply removed by peeling or otherwise removing it from the patient's dentition.

## Claims

1. A method of providing a combined sheet and tooth whitening material, said method comprising:
(a) providing a mixture comprising
- a powder phase comprising a salt selected from the group consisting of a mixed powder of sodium/calcium salts of the copolymer of methyl vinyl ether/maleic anhydride, a blend of mixed sodium/calcium salts of the copolymer of methyl vinyl ether/maleic anhydride, and mixtures thereof, and optionally, a filler; and
- a liquid phase comprising a solution of from 3 to 50 percent by weight of hydrogen peroxide in water;
(b) decanting the mixture into a mold before the mixture has set;
(c) allowing the mixture to at least partially set; and
(d) pressing a release or backing sheet (41) to the at least partially set material (40).

2. The method as in claim 1, wherein said filler is titanium dioxide or silica, and is present in an amount of from 0 to 10 percent by weight based upon the total weight of said mixture.

3. The method as in claim 1, wherein the mixture comprises from 10 to 70 percent by weight of said hydrogen peroxide based upon the total weight of said mixture.

4. The method as in claim 3, wherein said hydrogen peroxide is present in an amount of from about 30 to about 35 percent by weight salt based upon the total weight of said mixture.

5. The method as in claim 1, further comprising from 0 to 40 percent by weight of carbamide peroxide based upon the total weight of said mixture.

6. The method as in claim 1, further comprising from 0 to 30 percent by weight of glycerin based upon the total weight of said mixture.

7. The method as in claim 1, further comprising from 0 to 40 percent by weight of water based upon the total weight of said mixture.

8. The method as in claim 1, further comprising from 15 to 65 percent by weight of said salt based upon the total weight of said mixture.

9. The method as in claim 1, wherein said salt is Gantrez MS-955.

## Patentansprüche

1. Verfahren zur Bereitstellung eines kombinierten Folien- und Zahnbleichungsmaterials, wobei das Verfahren umfasst:
(a) Bereitstellung eines Gemisches, umfassend
- eine Pulverphase, umfassend ein Salz ausgewählt aus der Gruppe bestehend aus einem gemischten Pulver aus Natrium/Kalziumsalzen eines Copolymers aus Methylvinylether/Maleinsäureanhydrid, ein Blend aus gemischten Natrium/Kalziumsalzen eines Copolymers aus Methylvinylether/Maleinsäureanhydrid, und Gemischen davon, und gegebenenfalls ein Füllstoff; und
- eine flüssige Phase, umfassend eine Lösung aus 3 bis 50 Gewichtsprozent Wasserstoffperoxid in Wasser;
(b) Dekantierung des Gemisches in eine Form bevor das Gemisch ausgehärtet ist;
(c) zumindest teilweise Härtung des Gemisches; und
(d) aufpressen einer Folie (41) auf das zumindest teilweise ausgehärtete Material (40).

2. Das Verfahren nach Anspruch 1, wobei der Füllstoff Titandioxid oder Silica ist und in einer Menge von 0 bis 10 Gewichtsprozent bezogen auf das Gesamtgewicht des Gemisches vorliegt.

3. Das Verfahren nach Anspruch 1, wobei das Gemisch 10 bis 70 Gewichtsprozent Wasserstoffperoxid bezogen auf das Gesamtgewicht des Gemisches umfasst.

4. Das Verfahren nach Anspruch 3, wobei das Wasserstoffperoxid in einer Menge von 30 bis etwa 35 Gewichtsprozent des Salzes bezogen auf das Gesamtgewicht des Gemisches vorliegt.

5. Das Verfahren nach Anspruch 1, das weiterhin 0 bis 40 Gewichtsprozent Carbamidperoxid umfasst, bezogen auf das Gesamtgewicht des Gemisches.

6. Das Verfahren nach Anspruch 1, das weiterhin 0 bis 30 Gewichtsprozent Glyzerin bezogen auf das Gesamtgewicht des Gemisches umfasst.

7. Das Verfahren nach Anspruch 1, das weiterhin 0 bis 40 Gewichtsprozent Wasser bezogen auf das Gesamtgewicht des Gemisches umfasst.

8. Das Verfahren nach Anspruch 1, das weiterhin 15 bis 65 Gewichtsprozent des Salzes bezogen auf das Gesamtgewicht des Gemisches umfasst.

9. Das Verfahren nach Anspruch 1, wobei das Salz Gantrez MS-955 ist.

## Revendications

1. Procédé pour fournir une feuille et une matière de blanchiment dentaire combinées, ledit procédé comprenant les étapes consistant :
(a) à fournir un mélange comprenant
- une phase de poudre comprenant un sel choisi dans le groupe consistant en une poudre mixte de sels de sodium/calcium du copolymère éther méthylvinylique/anhydre maléique, un mélange de sels mixtes de sodium/calcium du copolymère éther méthylvinylique/anhydre maléique, et leurs mélanges, et, facultativement, une charge ; et
- une phase liquide comprenant une solution de 3 à 50 % en poids de peroxyde d'hydrogène dans de l'eau ;
(b) à décanter le mélange dans un moule avant la prise du mélange ;
(c) à laisser le mélange prendre au moins partiellement ; et
(d) à presser une feuille de séparation ou de renforcement (41) à ladite matière ayant pris au moins partiellement(40).

2. Procédé suivant la revendication 1, dans lequel ladite charge est le dioxyde de titane ou la silice et est présente en une quantité de 0 à 10 pour cent en poids sur la base du poids total dudit mélange.

3. Procédé suivant la revendication 1, dans lequel le mélange comprend 10 à 70 pour cent en poids dudit peroxyde d'hydrogène sur la base du poids total dudit mélange.

4. Procédé suivant la revendication 3, dans lequel ledit peroxyde d'hydrogène est présent en une quantité d'environ 30 à environ 35 pour cent en poids du sel sur la base du poids total dudit mélange.

5. Procédé suivant la revendication 1, comprenant en outre 0 à 40 pour cent en poids de peroxyde de carbamide sur la base du poids total dudit mélange.

6. Procédé suivant la revendication 1, comprenant en outre 0 à 30 pour cent en poids de glycérol sur la base du poids total dudit mélange.

7. Procédé suivant la revendication 1, comprenant en outre 0 à 40 pour cent en poids d'eau sur la base du poids total dudit mélange.

8. Procédé suivant la revendication 1, comprenant en outre 15 à 65 pour cent en poids dudit sel sur la base du poids total dudit mélange.

9. Procédé suivant la revendication 1, dans lequel ledit sel est le Gantrez MS-955.
